# EUROPEAN PATENT SPECIFICATION

(11) **EP 3 726 987 B1**
(45) Date of publication and mention of the grant of the patent: **19.03.2025**
(21) Application number: 18814964.5
(22) Date of filing: 13.12.2018
(51) Int. Cl.: A01N 31/02, A01N 31/08, A01N 31/16, A61K 8/34, A61Q 17/02, A61K 8/97, A01P 1/00

(54) **AN ANTIMICROBIAL COMPOSITION**
ANTIMIKROBIELLE ZUSAMMENSETZUNG
COMPOSITION ANTIMICROBIENNE

(30) Priority: 22.12.2017 EP 17209949
(43) Date of publication of application: 28.10.2020
(73) Proprietor: Unilever Global IP Limited, Wirral, Merseyside CH62 4ZD (GB); Unilever IP Holdings B.V., 3013 AL Rotterdam (NL)
(72) Inventor: DASGUPTA, Anindya, Whitefield, Bangalore 560 066 (IN); PATIL, Nivedita, Jagdish, Whitefield, Bangalore 560 066 (IN); SAJI, Maya, Treesa, Whitefield, Bangalore 560 066 (IN); WASKAR, Morris, Whitefield, Bangalore 560 066 (IN)
(74) Representative: van den Brom, Coenraad Richard
(86) International application number: PCT/EP2018/084749
(87) International publication number: WO 2019/121319

(56) References cited:
- WO-A1-01/82922
- WO-A1-2011/038797
- CN-A- 101 002 798
- CN-A- 102 716 188
- US-A- 5 532 290
- US-A1- 2017 173 162
- MOORER WR: "Antiviral activity of alcohol for surface disinfection", INT. DENT. HYGIENE, vol. 1, 1 January 2003 (2003-01-01), XP055834836
- G. KAMPF: "Efficacy of ethanol against viruses in hand disinfection", JOURNAL OF HOSPITAL INFECTION, vol. 98, no. 4, 1 April 2018 (2018-04-01), AMSTERDAM, NL, pages 331 - 338, XP055714645, ISSN: 0195-6701, DOI: 10.1016/j.jhin.2017.08.025
- MALIK ET AL: "Comparative efficacy of ethanol and isopropanol against feline calicivirus, a norovirus surrogate", AJIC: AMERICAN JOURNAL OF INFECTION CONTROL, ELSEVIER, AMSTERDAM, NL, vol. 34, no. 1, 1 February 2006 (2006-02-01), pages 31 - 35, XP005442120, ISSN: 0196-6553, DOI: 10.1016/J.AJIC.2005.05.012
- DATABASE CA [online] CHEMICAL ABSTRACTS SERVICE, COLUMBUS, OHIO, US; MATSUI, HIDEO ET AL: "Synthesis and electronic behaviors of alternating indium-organic moiety binary hybrid copolymer", XP002778749, retrieved from STN Database accession no. 2006:373319
- DATABASE CA [online] CHEMICAL ABSTRACTS SERVICE, COLUMBUS, OHIO, US; GLIDEWELL, CHRISTOPHER ET AL: "Supramolecular chemistry of amine-phenol adducts; novel three-dimensional framework structures in adducts of bis(2-aminoethyl)amine with 4,4'-sulfonyldiphenol, 1,1,1-tris(4-hydroxyphenyl)ethane and 3,5-dihydroxybenzoic acid, and in the methanol-solvated adduct of tris(2-aminoethyl)amine with 4,4'-bi", XP002778750, retrieved from STN Database accession no. 2000:176728
- DATABASE CA [online] CHEMICAL ABSTRACTS SERVICE, COLUMBUS, OHIO, US; GOLDBERG, DAVID P. ET AL: "A decanuclear mixed-valent manganese complex with a high spin multiplicity in the ground state", XP002778751, retrieved from STN Database accession no. 1993:616198

## Description

### Field of the invention

The present invention relates to an antimicrobial composition. Particularly, the present invention relates to an antiviral composition.

### Background of the invention

People try to take good care of external surfaces of their bodies as well as those of their pets to maintain good overall health. These surfaces include their skin, oral cavity and scalp. Specific skin related issues that people are concerned about include healthy and infection-free skin, good skin tone and adequate moisturization. Oral cavity is another external surface that people actively take care to maintain. People generally prefer thick long hair with minimum hair fall and an infection-free scalp.

Many if not all problems related to skin, oral cavity or scalp, are linked to microorganisms like e.g. bacteria, fungi and viruses. For example, some bacteria like *Escherichia coli (E. coli)* and *Staphylococcus aureus (S. aureus)* are commonly found on the human skin. These bacteria *per se* are not pathogenic whilst being commonly present on the skin. However, when they enter the human body through acts like ingestion, these bacteria tend to become pathogenic. Much of problems of the oral cavity including gums and teeth, like e.g. cavities, tartar, gingivitis, caries, bad breath also known as halitosis and plaque are associated with presence of bacteria in the oral cavity. Dandruff, a commonly occurring scalp problem too, is an implication of scalp infected by a fungal microorganism.

In addition, people also get exposed to viruses. Viruses, in general, are of two types non-enveloped like e.g. rotavirus and adenovirus, or of enveloped type like e.g. hepatitis B virus and respiratory syncytial virus. In enveloped viruses, an outer lipid membrane is present whereas the membrane is absent in non-enveloped viruses. Due to this difference, it is relatively easier to obtain antiviral activity against enveloped viruses as it is relatively easy to make the outer lipid membrane rupture upon application of antimicrobial actives. Whereas, for obtaining an antiviral activity against non-enveloped viruses an antimicrobial active need to act on proteins and nucleic acids contained in a virus. For this reason, obtaining antiviral activity against non-enveloped viruses has remained a challenge. A reduction in virus titer of even 1 log in case of non-enveloped virus is said to be a significant benefit.

People often encounter common cold which is caused by viruses like e.g. adenovirus. Further, norovirus is a common cause of gastroenteritis in humans. Norovirus is said to have similarity to feline calicivirus (FCV). Due to this similarity, FCV is widely used in research. For example, studies have been carried out where effectiveness of handwashing is estimated based on FCV removal.

As microorganisms like bacteria, viruses and fungi, at times, give rise to unwanted effects, people often tend to get rid of or minimize exposure to microorganisms. For this, they take care of their external surfaces that includes skin like e.g. skin of their hands, oral cavity and scalp, by keeping these surfaces free of microorganisms. One way to tackle infections is to treat them with antimicrobial actives after the infection has set in. Whereas, another approach is to leave on these surfaces a minimal amount of antimicrobial active so that any invading microorganisms are killed or inactivated thereby minimizing spread of an infection.

A few references are found in prior art that relate to certain actives and antimicrobial benefit obtained therefrom.

WO11038797 (Beiersdorf AG, 2009) discloses use of magnolol or honokiol as antibacterial, antimycotic, antiparasitic or antiviral active ingredients, wherein only one of the two active ingredients are used in each case.

US2009156551 (DSM, 2009) discloses compositions comprising magnolol and honokiol wherein the mol ratio of magnolol to honokiol is less than 0.6 as well as to the use of these compositions as medicament, in particular as a medicament for the treatment, co-treatment or prevention of inflammatory disorders.

WO0182922 (Procter and Gamble, 2001) discloses oral compositions comprising an effective amount of a polyphenol herbal extract selected from the group consisting of magnolol, honokiol, tetrahydromagnolol, tetrahydrohonokiol, and mixtures thereof; an effective amount of a buffering agent; from about 40 percent to about 99 percent of one or more aqueous carriers; wherein the oral composition has a total water content of from about 5 percent to about 70 percent.

CN101002798 (Guo Zijie) discloses a medicine in the form of spray for preventing and treating beriberi and its complications wherein the composition comprises gamba, from 0.01 to 9% magnolol, menthol, borax, from 3 to 45% ethanol, urea and purified water. The composition relates to cool sterilization foot sprays.

US 2017/173162 A1 relates to compositions that can act as preservatives, antimicrobial agents, or a combination thereof, including embodiments directed to preservatives derived from extracts that are derived from Albizia amara seeds and Magnolia officinalis barks.

CN 102 716 188 A relates to a method for extracting an extract from Mangnolia officinalis fruit and the antibacterial application thereof.

US 5 532 290 A relates to antimicrobial polymers and compositions containing them.

Y.S. Malik et al., American Journal of Infection Control Vol 34, No. 1 (2006), 31-35, doi: 10.1016/j.ajic.2005.05.012) discuss the comparative efficacy of ethanol and isopropanol against feline calicivirus.

W.R. Moorer, International Journal of Dental Hygiene, Vol 1, No 1 (2003), 128-142, reviews the antiviral activity of alcohol for surface disinfection.

Despite efforts thus far, people are always on a look out for new technologies for delivering antimicrobial benefit. Therefore, antimicrobial compositions and actives that deliver an antimicrobial benefit, remains a topic of interest.

Need therefore exists to provide an antimicrobial composition comprising one or more actives, that deliver an antimicrobial benefit.

It is an object of the present invention to provide an antimicrobial composition for combating common disease causing viruses.

It is another object of the present invention to provide a - possibly synergistic - antimicrobial benefit, particularly; a - possibly synergistic - antiviral benefit, in relatively shorter contact times.

It is another object of the present invention to provide an antiviral composition. The present inventors have surprisingly found that a combination of at least one compound selected from biphenol and monohydric non-phenolic alcohol as defined in the claims provides a - possibly synergistic - antimicrobial benefit. Particularly, it may provide synergistic antiviral benefit. Moreover, it provides such benefit in relatively shorter contact times, e.g. 15 seconds. It has also been found that such antimicrobial benefit is obtained in relatively short contact times against gram positive and gram-negative microorganisms.

### Summary of the invention

In a first aspect, the present invention relates to an antimicrobial composition comprising:
a. from 0.1 to 4% by weight at least one biphenol selected from honokiol, magnolol or unsubstituted 2,2'-biphenol; and
b. from 60 to 80% by weight monohydric non-phenolic alcohol.
wherein the alcohol is selected from ethyl alcohol, isopropyl alcohol and mixtures thereof.

In a second aspect, the present invention relates to a non-therapeutic method of cleaning or disinfecting a surface comprising the step of applying on to a surface a composition of the first aspect.

In a third aspect, the present invention relates to non-therapeutic use of a composition of the first aspect for antimicrobial benefit.

In a fourth aspect, the present invention relates to non-therapeutic use of a composition of the first aspect for improved hand hygiene.

### Detailed description of the invention

Any feature of one aspect of the present invention may be utilized in any other aspect of the invention. The word "comprising" is intended to mean "including" but not necessarily "consisting of" or "composed of." In other words, the listed steps or options need not be exhaustive Numerical ranges expressed in the format "from x to y" are understood to include x and y. When for a specific feature multiple preferred ranges are described in the format "from x to y", it is understood that all ranges combining the different endpoints are also contemplated.

In a first aspect, the present invention relates to an antimicrobial composition comprising:
a. from 0.1 to 4% by weight at least one biphenol selected from honokiol, magnolol or unsubstituted 2,2'-biphenol; and
b. from 60 to 80% by weight monohydric non-phenolic alcohol.
wherein the alcohol is selected from ethyl alcohol, isopropyl alcohol and mixtures thereof.

Preferably, an antimicrobial composition according to the present invention can be an antiviral composition, antibacterial composition, antifungal composition. More preferably, an antimicrobial composition according to the present invention can be an antiviral composition or an antibacterial composition. Most preferably, an antimicrobial composition according to the present invention is an antiviral composition.

### Biphenol

The composition as per the present invention (the composition) comprises at least one biphenol selected from honokiol (5,3'-Diallyl-2,4'-dihydroxybiphenyl), magnolol (5,5'-Diallyl-2,2'-dihydroxybiphenyl) or unsubstituted 2,2'-biphenol.

The composition comprises from 0.1% to 4% by weight of the biphenol.

### Monohydric non-phenolic alcohol

The composition also comprises a non-phenolic alcohol. The alcohol is selected from ethyl alcohol, isopropyl alcohol and mixtures thereof.

Preferably, the composition comprises from 60 to 80%, more preferably from 60 to 75%, even more preferably from 60 to 72%, still more preferably 60 to 70%, yet more preferably from 61 to 68% and most preferably from 62 to 65% by weight of the non-phenolic monohydric alcohol.

It is required that an amount of the monohydric non-phenolic alcohol that is present in the composition does not exceed 80% by weight of the composition. When monohydric non-phenolic alcohol e.g. ethanol, is used in amounts greater than 80%, it tends to evaporate faster and is said to be less efficacious on substrate it is applied on to. In addition, such high amounts of the alcohol are also said to be associated with skin dryness and irritation.

### Surfactants

Preferably, the composition further comprises surfactants to provide cleaning benefit. Preferably, the surfactants are selected from anionic, nonionic, cationic, amphoteric surfactants and mixtures thereof. More preferably, surfactants are selected from cationic surfactant, nonionic surfactant and mixtures thereof. Most preferably, surfactants are selected from nonionic surfactants.

Preferred anionic surfactants include soap, alkyl ether sulfate and sulfonates, alkyl sulfates and sulfonates, alkylbenzene sulfonates, alkyl and dialkyl sulfosuccinates, C₈-C₂₀ acyl isethionates, acyl glutamates, C₈-C₂₀ alkyl ether phosphates and mixtures thereof.

Preferred nonionic surfactants are those with a C₁₀-C₂₀ fatty alcohol or acid hydrophobe condensed with from 2 to 100 moles of ethylene oxide or propylene oxide per mole of hydrophobe; C₂-C₁₀ alkyl phenols condensed with from 2 to 20 moles of alkylene oxide; mono- and di- fatty acid esters of ethylene glycol; fatty acid monoglyceride; sorbitan, mono- and di- C₈-C₂₀ fatty acids; block copolymers (ethylene oxide/propylene oxide); polyoxyethylene sorbitan and mixtures thereof. Alkyl polyglycosides and saccharide fatty amides (e.g. methyl gluconamides) are also suitable nonionic surfactants.

Preferred cationic surfactant include cetyl trimethylammonium bromide, benzalkonium halides that are also also known as alkyldimethylbenzylammonium halides. Preferred cationic surfactant that may be used in the composition is benzalkonium chloride, also known as alkyldimethylbenzylammonium chloride.

Preferred amphoteric surfactant include amide, betaine and amine oxide type. Particularly amphoteric surfactants include cocodiethanol amide and cocomonoethanol amide, cocoamidopropyl betaine and coco amido propyl amine oxide. A preferred amphoteric surfactant that may be used as a surfactant in the composition is cocoamidopropyl betaine.

When incorporated in the composition, surfactants may be present in an amount from 1 to 80%, preferably from 3 to 60%, more preferably from 5 to 40%, even more preferably from 7 to 30%, further more preferably from 9 to 15 % by weight of the composition.

Preferably, the composition further comprises a cosmetically acceptable base. The cosmetically acceptable base is preferably in the form of a cream, lotion, gel or emulsion.

The compositions may be prepared using different cosmetically acceptable emulsifying or non-emulsifying base. A highly suitable base is a cream. Vanishing creams are especially preferred. Vanishing cream bases generally comprise a mixture of fatty acid and soap. Vanishing cream base gives a highly appreciated matty feel to the skin. C12 to C20 fatty acids are especially preferred in vanishing cream bases, further more preferred being C14 to C18 fatty acids. Most preferably, the fatty acids are stearic acid or palmitic acid or mixtures thereof. The fatty acid is often hystric acid which is substantially (generally from 90 to 95% by weight) a mixture of 45% stearic and 55% palmitic acid. Thus, inclusion of hystric acid and its soap to prepare compositions of the invention is within the scope of the present invention. The fatty acid in the composition is preferably present in an amount from 5 to 20%, more preferably from 6 to 19% and even more preferably from 7 to 17% by weight of the composition. Soaps in the vanishing cream base include alkali metal salt of fatty acids, like sodium or potassium salts, most preferred being potassium stearate. The soap in the vanishing cream base is generally present in an amount in the range from 0.1 to 10%, more preferably from 0.1 to 3% by weight of the composition. Generally, the vanishing cream base topical compositions are prepared by taking a desired amount of total fatty matter and mixing with potassium hydroxide in desired amounts. The soap is usually formed in-situ during the mixing.

An especially suitable cosmetically acceptable base is one which comprises a water-in-oil emulsion comprising silicone oils as the continuous phase. The water-in-oil emulsions preferably comprise a cross-linked silicone elastomer blend.

Inclusion of silicone elastomer blend in a water-in-oil emulsion may be used as the cosmetically acceptable base for preparing the compositions of the present invention. While silicone fluids may be used, silicone elastomers which are cross-linked, are especially preferred. In contrast to silicone fluid polymers, the physical properties of elastomers are typically dependent on the number of cross-linkages, rather than molecular weight. The ability of silicone elastomers to swell makes them ideal thickeners for oil phases. The elastomers have a very smooth and soft feel when applied to skin or hair. They can also be used as delivery agents for fragrances, vitamins and other additives in cosmetic compositions. Suitable silicone elastomer blends or gels which are commercially available and suitable for inclusion in the composition of the invention and found to provide the enhanced stability are: Dow Corning^{®} EL-8051 IN Silicone Organic Elastomer Blend [INCI Name: Isodecyl Neopentanoate (and) Dimethicone/Bis Isobutyl PPG-20 Crosspolymer], EL-8050 [INCI Name: Isododecane (and) Dimethicone/Bis-Isobutyl PPG 20 Crosspolymer], DC9040, DC9041, DC9045 (Dimethicone crosspolymer), DC9506, DC9509 (Dimethicone vinyl dimethicone crosspolymer) and Shin-Etsu KSG-15, KSG-16, KSG-17 (Dimethicone vinyl dimethicone crosspolymer). It is further preferred that the composition comprises from 5 to 50% by weight silicone elastomer.

Water and/or polyhydric alcohol may also be used a cosmetically acceptable base. Polyhydric alcohols commonly comprise ethylene or propylene glycol, or a homologue can be employed such as diethylene glycol.

Preferably, the cosmetically acceptable base is present preferably from 10 to 99.9%, more preferably from 50 to 99%, even more preferably from 60 to 85% and furthermore preferably from 65 to 80% by weight of the composition.

Preferably, the composition further comprises skin lightening agents. Examples of skin lightening agents that may be used in the composition include, 12-hydroxystearic acid, aloe extract, ammonium lactate, arbutin, azelaic acid, kojic acid, butyl hydroxy anisole, butyl hydroxy toluene, citrate esters, , 2, 5 dihydroxybenzoic acid, ellagic acid, fennel extract, glucopyranosyl-1-ascorbate, gluconic acid, glycolic acid, green tea extract, hydroquinone, 4 hydroxyanisole, hydroxycaprylic acid, lemon extract, linoleic acid, magnesium ascorbyl phosphate, mulberry root extract, salicylic acid, vitamins like vitamin B6, vitamin B12, vitamin C, vitamin A, a dicarboxylic acid, hydroxycarboxylic acid like lactic acid and their salts e.g. sodium lactate and mixtures thereof.

Preferably, the composition further comprises one or more sunscreens. Any sunscreen that can be suitably used with the base may be added. Both, UVA and UVB sunscreens may preferably be added.

The composition of the invention may preferably comprise a UV- A sunscreen which is a dibenzoylmethane or its derivatives. Preferred dibenzoylmethane derivatives are selected from 4-tert-butyl-4'-methoxydibenzoylmethane, 2-methyldibenzoylmethane, 4-methyl-dibenzoylmethane, 4-isopropyldibenzoyl-methane, 4-tert-butyldibenzoylmethane, 2,4-dimethyldibenzoylmethane, 2,5-dimethyldibenzoylmethane, 4,4'-diisopropyl-dibenzoylmethane, 2-methyl-5-isopropyl-4'-methoxydibenzoylmethane, 2-methyl-5-tert-butyl-4'-methoxydibenzoyl methane, 2,4-dimethyl-4'- methoxy dibenzoylmethane or 2,6-dimethyl-4-tert-butyl-4'-methoxy-dibenzoylmethane. The most preferred dibenzoylmethane derivative is 4-tert-butyl-4'-methoxydibenzoylmethane. The composition of the invention preferably comprises from 0.1 to 10%, more preferably from 0.2 to 5%, furthermore preferably from 0.4 to 3%, by weight dibenzoylmethane or said derivatives based on total weight of the composition and including all ranges subsumed therein.

The composition may also preferably comprise a UV-B organic sunscreen selected from the class of cinnamic acid, salicylic acid and diphenyl acrylic acid. Illustrative non-limiting example of UV-B sunscreens which are commercially available and useful for inclusion in the composition of the invention are Octisalate^{™}, Homosalate^{™}, NeoHelipan^{™}, Octocrylene^{™}, Oxybenzone^{™} or Parsol MCX^{™}. The UV-B sunscreen is most preferably 2-ethyl-hexyl-4-methoxy cinnamate which is commercially available as Parsol MCX^{™}. The UV-B organic sunscreen is preferably included in the composition from .1 to 10%, more preferably from 0.1 to 7 % by weight of the composition. It has been observed that presence of an organic UV-B sunscreen like 2-ethyl-hexyl-4-methoxy cinnamate causes further rapid degradation of the UV-A dibenzoylmethane sunscreen in the presence of UV radiation. The presence of the rosmarinic acid ester compound is found to be very efficacious in stabilizing the composition even when UV-B sunscreens are present.

Useful inorganic sun-blocks are also preferably used in the present invention. These include, for example, zinc oxide, iron oxide, silica, such as fumed silica, and titanium dioxide.

The composition may further comprise preservatives to protect against the growth of potentially harmful microorganisms. Suitable traditional preservatives for compositions of this invention are alkyl esters of para-hydroxybenzoic acid. Other preservatives which have more recently come into use include propionate salts. Particularly preferred preservatives are phenoxyethanol, methyl paraben, propyl paraben, imidazolidinyl urea, sodium dehydroacetate, benzyl alcohol, alkane diols most preferably 1,2-octane diol and phenoxyethanol. The preservatives should be selected having regard for the use of the composition and possible incompatibility between the preservatives and other ingredients. Preservatives are preferably employed in amounts from 0.01% to 2% by weight of the composition.

A variety of other optional materials may be formulated into the compositions. These may include antimicrobials such as 2-hydroxy-4,2',4'-trichlorodiphenylether (triclosan), 2,6-dimethyl-4-hydroxychlorobenzene, and 3,4,4'-trichlorocarbanilide, scrub and exfoliating particles such as polyethylene and silica or alumina; cooling agents such as menthol; skin calming agents such as aloe vera; and colorants.

In addition, the compositions may further comprise from 0 to 10% by weight of opacifiers and pearlizers such as ethylene glycol distearate, titanium dioxide or Lytron^{®} 621 (Styrene/Acrylate copolymer); all of which are useful in enhancing the appearance or properties of the product.

Diluents other than water that may be used in the composition includes liquid or solid emollients, solvents, humectants, thickeners and powders. Examples of each of these types of vehicle, which can be used singly or as mixtures of one or more vehicles, are as follows:

Preferably, the composition comprises emollients. Examples of emollients that may be present include stearyl alcohol, glyceryl monoricinoleate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rape seed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isostearic acid, palmitic acid, isopropyl linoleate, lauryl lactate, myristyl lactate, decyl oleate, myristyl myristate.

Preferably, the composition comprises solvents such as acetone, ethylene glycol monoethyl ether, diethylene glycol monobutyl ether, diethylene glycol monoethyl ether and mixtures thereof.

Advantageously, the composition may preferably comprise ingredients like bactericides, vitamins, anti-acne actives, anti-wrinkle, anti-skin atrophy and skin repair actives, skin barrier repair actives, non-steroidal cosmetic soothing actives, artificial tanning agents and accelerators, sebum stimulators, sebum inhibitors, anti-oxidants, protease inhibitors, skin tightening agents, anti-itch ingredients, hair growth inhibitors, 5-alpha reductase inhibitors, desquamating enzyme enhancers, anti-glycation agents and mixtures thereof.

The composition may preferably comprise powders like e.g. chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate and mixtures thereof.

The compositions of the present invention can comprise a wide range of other optional components. The CTFA Personal care Ingredient Handbook, Second Edition, 1992 describes a wide variety of non-limiting personal care and pharmaceutical ingredients commonly used in the skin care industry, which are suitable for use in the compositions of the present invention. Examples include: antioxidants, binders, biological additives, buffering agents, colorants, thickeners, polymers, astringents, fragrance, humectants, opacifying agents, conditioners, pH adjusters, natural extracts, skin sensates, skin soothing agents, and skin healing agents.

The composition may also comprise one or more of the following ingredients e.g. chloroxylenol, zinc pyrithione (ZPT), creatine and creatinine.

The composition is preferably in the form of a wash-off or a leave-on composition, preferably a leave-on composition.

Wash-off composition preferably means composition which is intended/required to be removed from the body by washing with solvent preferably water after the application of the composition like e.g. hand wash composition and face wash composition.

Leave-on composition preferably means composition which is not required to be removed from the human body after the application of the composition like e.g. skin cream, body lotion, hand sanitizer and deodorants.

When the composition is in the form of a leave-on composition, the composition may be in the form of a deodorant, a hand sanitizer, a lotion, a cream and a body spray.

The composition of the invention may preferably comprise a conventional deodorant base as the cosmetically acceptable carrier. By a deodorant is meant a product in the stick, roll-on, or propellant medium which is used for personal deodorant benefit e.g. application in the under-arm area which may or may not contain anti-perspirant actives.

Deodorant compositions can generally be in the form of firm solids, soft solids, gels, creams, and liquids and are dispensed using applicators appropriate to the physical characteristics of the composition. Deodorant compositions which are delivered through roll-ons generally comprise a liquid carrier. Such liquid carrier can be hydrophobic or comprise a mixture of both hydrophilic and hydrophobic liquids. They may be in the form of an emulsion or a microemulsion. The liquid carrier or mixture of carriers often constitutes from 30 to 95% and in many instances from 40 to 80% by weight of the composition. Hydrophobic liquid carriers commonly can comprise one or more materials selected within the chemical classes of siloxanes, hydrocarbons, branched aliphatic alcohols, esters and ethers that have a melting point not higher than 25°C and a boiling point of at least 100°C. Hydrophilic carrier liquids that can be employed in compositions herein commonly comprise water and/or a polyhydric alcohol or water-miscible homologue. Polyhydric alcohols commonly comprise ethylene or propylene glycol, or a homologue can be employed such as diethylene glycol. Other than this suitable other vehicle and component used for deodorant composition can be added.

In a second aspect, the present invention also provides a non-therapeutic method of cleaning or disinfecting a surface comprising the steps of applying the composition of the first aspect on to a surface in case of a leave-on composition. This method optionally comprises an additional step of at least partially removing the composition from the surface if it is in the form of a wash-off composition. Preferably, the step of at least partially removing the composition is carried out in less than 5 minutes after the step of applying the composition on to the surface. The method is non-therapeutic.

In a third aspect, the present invention also provides non-therapeutic use of the composition of the first aspect for antimicrobial benefit. An antimicrobial benefit preferably means after application of the composition, the residual microbes on the surface is significantly less. The composition of the present invention provides improved antimicrobial benefits. Particularly, it provides a - possibly synergistic - antiviral benefit.

In a fourth aspect, the present invention also provides non-therapeutic use of the composition for improved hand hygiene.

The present invention now will be demonstrated by way of following non-limiting examples.

### Examples

### In-vitro experiments to demonstrate antimicrobial efficacy of the composition as per the present invention assessed using log reduction of Adenovirus:

### Materials:

Human adenovirus 5; Strain- Adenoid 75 (ATCC-VR-5),
Feline Calicivirus (FCV; F-9 strain from ATCC),
Crandell Rees Feline Kidney cells (CRFK; CCL-94 from ATCC),
HeLa cells (CCL-23 from ATCC),
Honokiol (98% pure from World Way Biotech, China),
Magnolol (98% pure from World Way Biotech, China),
2,2'-Biphenol (Aldrich cat. No. 115819),
Sephacryl column MicroSpin^{™} S-400 (GE Healthcare Cat# 27-5140-01),
Dulbecco's modified Eagel's medium (DMEM; Sigma cat. No. D6046),
Fetal calf serum (FCS; Gibco cat. No. 16000-044),
Virus Growth Medium (VGM, DMEM + 2% FCS),
Ethylene diamine tetra acetic acid (EDTA; Sigma cat. No. E6758); and
4-(2-hydroxyethyl)-1-piperazineethanesulfonic acid (HEPES; Sigma cat. No. H3375).

The experiment was carried out using the following protocol:
The antiviral effectiveness was tested as per ASTM International test method designated E1052-11 "Standard Test Method to Assess the Activity of Microbicides against Viruses in Suspension".

1 mL of compositions outlined in table 1 were aliquoted in sterile microfuge tubes. To all these tubes, 50 µL of high titer virus stocks of adenovirus was added, mixed and held for 15 seconds at 24°C. After this, 300 µL of this mixture was added to 300 µL of neutralizer (DMEM + 10% FCS + 5% HEPES + 1mM EDTA) and was vortexed. 100 µL of each neutralized mixture was added on sephacryl column and centrifuged at 735g for 2 min at 4°C. The virus containing elute was serially diluted in VGM and added on HeLa in case of adenovirus. Monolayer of HeLa cells were grown in 96-well cell culture plates at 37°C under 5% CO₂.

**Table 1:**

| Example | Water | Sanitizer Base^{#} | Honokiol | Magnolol | 2,2' - Biphenol |
|---|---|---|---|---|---|
| A | 100 | - | - | - | - |
| B | 99.5 | - | 0.25 | 0.25 | - |
| C | - | 100 | - | - | - |
| 1 | - | 99.5 | 0.5 | - | - |
| 2 | - | 99.75 | 0.25 | - | - |
| 3 | - | 99.75 | 0.125 | 0.125 | - |
| 4 | - | 99.5 | - | - | 0.5 |
| 5 | - | 99.5 | 0.25 | 0.25 | - |

| | | | | | |
|---|---|---|---|---|---|
| ^{#}Sanitizer base comprises 62% ethyl alcohol, 3% isopropyl alcohol, minors like polymer and perfume up to 0.5% and water up to 100%. | | | | | |

100 µL of each dilution prepared as mentioned above were added in five replicate wells in 96-well plate containing HeLa cells. In addition, each plate had cells along with VGM serving as cells control. All the plates were incubated at 37°C under 5% CO₂ and observed daily for virus specific cytopathic effect for the period up to 5 days. At the end of the incubation period (5 days) TCID50 (Tissue Culture Infective Dose, virus titer) was calculated using Spearman-Kärber formula as described in annexure C of BS EN 14476:2013. The results were tabulated in table 2 below in terms of Log reduction in virus count.

**Table 2**

| Example | Adenovirus titer (TCID50) | Log reduction of Adenovirus at 15 seconds |
|---|---|---|
| A | 5.5 ± 0.24 | - |
| B | 5.5 ± 0.24 | 0 |
| C | 5.1 ± 0.35 | 0.4 |
| 1 | 4.1 ±0.0 | 1.4 |
| 2 | 4.1 ±0.0 | 1.4 |
| 3 | 4.1 ±0.0 | 1.4 |
| 4 | 4.5 ±0.0 | 1 |
| 5 | 3.6 ± 0.34 | 1.9 |

The data for example A in table 2 above shows adenovirus titer to be 5.5 ± 0.24 in case of control. This is taken to be the virus titer obtained after treatment only with water (example A; control). The log reduction in adenovirus titer for all the other examples (examples B, C and 1 to 5) were calculated by subtracting TCID50 values of these examples from TCID50 of example A.

The data in table 2 above shows that examples (Example 1 to 5) those are within the scope of the present invention provides much higher log reduction when compared with examples (Example A to C) that are outside the scope of the present invention.

The data further shows that log reduction of 0.4 in adenovirus titer was obtained due to treatment with sanitizer base alone (example C). However, upon treatment with the composition as per the present invention (Examples 1 to 7), almost 1 log reduction in titer of adenovirus, a non-enveloped virus, is obtained.1 log reduction in virus count of a non-enveloped virus is considered to very effective.

### In-vitro experiments to demonstrate antimicrobial benefit of the composition as per the present invention assessed using log reduction of FCV:

The protocol for this experiment was the same as disclosed in the previous section for adenovirus. However, in case of FCV the CRFK cells were used instead of HeLa and the incubation time was 2 days instead of 5 days.

Antimicrobial benefit for treatments outlined in table 3 below was estimated using the protocol described earlier.

**Table 3**

| Example | Water | Sanitizer Base | Honokiol | Magnolol |
|---|---|---|---|---|
| D | 100 | - | - | - |
| E | 99.5 | - | 0.25 | 0.25 |
| F | - | 99.5 | - | - |
| 6 | - | 99.5 | 0.5 | - |
| 7 | - | 99.5 | 0.25 | 0.25 |

The resulting log reduction in titer of FCV was tabulated in table 4 below.

**Table 4**

| Example | FCV titer (TCID50) | Log reduction FCV at 15 seconds |
|---|---|---|
| D | 5.6 ±0.2 | - |
| E | 5.6 ± 0.2 | 0 |
| F | 5.4 ± 0.1 | 0.2 |
| 6 | 4.5 ±0.0 | 1.1 |
| 7 | 4.2 ± 0.6 | 1.4 |

The TCID50 for example D (control) was found to be 5.6 ± 0.2. This is the virus titer found after treatment only with water, i.e. control. The log reduction in titer of FCV for examples E, F, 6 and 7 was calculated by subtracting TCID50 of these examples from TCID50 of example D.

The data in table 4 shows that the composition as per the present invention comprising an alcohol and honokiol (Examples 6) showed almost 1 log reduction in FCV, a non-enveloped virus. This antimicrobial benefit was found to be further enhanced when magnolol was used in addition to honokiol along with an alcohol as shown in example 7.

### Additional in-vitro experiment to demonstrate antimicrobial efficacy of the composition outside the scope of the present invention assessed using log reduction of Adenovirus:

The protocol for this experiment was the same as disclosed in the previous section for adenovirus.

**Table 5**

| Example | Water | Sanitizer Base | Honokiol | Magnolol |
|---|---|---|---|---|
| G | 100 | - | - | - |
| H | 27 | 72.5 | 0.25 | 0.25 |

| | | | | |
|---|---|---|---|---|
| Note: Ethanol content in the composition of example H is 45% and isopropanol content is 2.1%. | | | | |

**Table 6**

| Example | Adenovirus titer (TCID50) | Log reduction FCV at 15 seconds |
|---|---|---|
| G | 5.9 | - |
| H | 5.3 | 0.6 |

The TCID50 for example G was found to be 5.9. This is the virus titer found after treatment only with water, i.e. control. The log reduction in titer of Adenovirus for example H was calculated by subtracting TCID50 of example H from TCID50 of example D.

The data in table 6 shows that when ethanol is combined in an amount not according to the present invention, with biphenol, i.e. honokiol and magnolol, in as shown in example **H,** log reduction in Adenovirus titer was found to be as low as 0.6, which is less than 1 log reduction. That is, no synergistic antiviral benefit was obtained.

In conclusion, a composition comprising at least one biphenol and at least one monohydric non-phenolic alcohol in certain amounts according to the invention, provides a - possibly synergistic - antimicrobial benefit, particularly; it provides a - possibly synergistic - antiviral benefit.

## Claims

1. An antimicrobial composition comprising:
a. from 0.1 to 4% by weight at least one biphenol selected from honokiol, magnolol or unsubstituted 2,2'-biphenol; and
b. from 60 to 80% by weight monohydric non-phenolic alcohol
wherein the alcohol is selected from ethyl alcohol, isopropyl alcohol and mixtures thereof.

2. A composition as claimed in claim 1 further comprising from 1 to 80% by weight of a surfactant.

3. A composition as claimed in claims 1 or 2 further comprising a cosmetically acceptable base.

4. A composition as claimed in any one of claims 1 to 3 wherein the composition is in the form of a wash-off composition.

5. A composition as claimed in any one of claims 1 to 4 wherein the composition is in the form of a leave-on composition.

6. A non-therapeutic method of cleaning or disinfecting a surface comprising the step of applying a composition as claimed in any one of claims 1 to 5 on to a surface.

7. A non-therapeutic method as claimed in claim 6 wherein the composition is in the form of a wash-off composition and wherein there is an additional step of at least partially removing the applied composition.

8. A non-therapeutic method as claimed in claim 7 wherein the step of at least partially removing the composition is carried out in less than 5 minutes after the step of applying the composition on to the surface.

9. Non-therapeutic use of a composition as claimed in any one of claims 1 to 5 for antimicrobial benefit.

10. Non-therapeutic use of a composition as claimed in any one of claims 1 to 5 for improved hand hygiene.

## Patentansprüche

1. Antimikrobielle Zusammensetzung, umfassend:
a. 0,1 bis 4 Gewichts-% mindestens eines Biphenols, ausgewählt unter Honokiol, Magnolol oder unsubstituiertem 2,2'-Biphenol; und
b. 60 bis 80 Gewichts-% einwertigen nicht-phenolischen Alkohol,
wobei der Alkohol unter Ethylalkohol, Isopropylalkohol und Mischungen davon ausgewählt ist.

2. Zusammensetzung, wie im Anspruch 1 beansprucht, die außerdem 1 bis 80 Gewichts-% eines Tensids umfasst.

3. Zusammensetzung, wie in den Ansprüchen 1 oder 2 beansprucht, die außerdem eine kosmetisch akzeptable Grundlage umfasst.

4. Zusammensetzung, wie in irgendeinem der Ansprüche 1 bis 3 beansprucht, wobei die Zusammensetzung in Form einer abwaschbaren Zusammensetzung vorliegt.

5. Zusammensetzung, wie in irgendeinem der Ansprüche 1 bis 4 beansprucht, wobei die Zusammensetzung in Form einer Leave-on-Zusammensetzung vorliegt.

6. Nicht-therapeutisches Verfahren zum Reinigen oder Desinfizieren einer Oberfläche, das den Schritt des Auftragens einer Zusammensetzung, wie in irgendeinem der Ansprüche 1 bis 5 beansprucht, auf eine Oberfläche umfasst.

7. Nicht-therapeutisches Verfahren, wie im Anspruch 6 beansprucht, wobei die Zusammensetzung in Form einer abwaschbaren Zusammensetzung vorliegt und wobei ein zusätzlicher Schritt zum zumindest teilweisen Entfernen der aufgetragenen Zusammensetzung durchgeführt wird.

8. Nicht-therapeutisches Verfahren, wie im Anspruch 7 beansprucht, wobei der Schritt des zumindest teilweisen Entfernens der Zusammensetzung in weniger als 5 Minuten nach dem Schritt des Auftragens der Zusammensetzung auf die Oberfläche durchgeführt wird.

9. Nicht-therapeutische Verwendung einer Zusammensetzung, wie in irgendeinem der Ansprüche 1 bis 5 beansprucht, mit antimikrobiellem Nutzen.

10. Nicht-therapeutische Verwendung einer Zusammensetzung, wie in irgendeinem der Ansprüche 1 bis 5 beansprucht, mit verbesserter Handhygiene.

## Revendications

1. Composition antimicrobienne comprenant :
a. de 0,1 à 4 % en poids d'au moins un biphénol choisi parmi l'honokiol, le magnolol ou un 2,2'-biphénol non substitué ; et
b. de 60 à 80 % en poids d'un alcool non phénolique monohydrique
dans laquelle l'alcool est choisi parmi l'éthanol, l'isopropanol et des mélanges de ceux-ci.

2. Composition selon la revendication 1 comprenant en outre de 1 à 80 % en poids d'un tensioactif.

3. Composition selon les revendications 1 ou 2 comprenant en outre une base cosmétiquement acceptable.

4. Composition selon l'une quelconque des revendications 1 à 3 dans laquelle la composition est sous la forme d'une composition à rincer.

5. Composition selon l'une quelconque des revendications 1 à 4 dans laquelle la composition est sous la forme d'une composition sans rinçage.

6. Procédé non thérapeutique de nettoyage ou de désinfection d'une surface comprenant l'étape d'application d'une composition selon l'une quelconque des revendications 1 à 5 sur une surface.

7. Procédé non thérapeutique selon la revendication 6 dans lequel la composition est sous la forme d'une composition à rincer et dans lequel il existe une étape supplémentaire d'élimination au moins partiellement de la composition appliquée.

8. Procédé non thérapeutique selon la revendication 7 dans lequel l'étape d'élimination au moins partiellement de la composition est réalisée en moins de 5 minutes après l'étape d'application de la composition sur la surface.

9. Utilisation non thérapeutique d'une composition selon l'une quelconque des revendications 1 à 5 pour un bénéfice antimicrobien.

10. Utilisation non thérapeutique d'une composition selon l'une quelconque des revendications 1 à 5 pour une hygiène des mains améliorée.
